# EUROPEAN PATENT SPECIFICATION

(11) **EP 0 640 589 B1**
(45) Date of publication and mention of the grant of the patent: **12.03.1997**
(21) Application number: 94112761.5
(22) Date of filing: 16.08.1994
(51) Int. Cl.: C07C 315/04, C07C 317/08

(54) **Process for manufacturing compounds containing two or more vinyl sulfone groups**
Verfahren zur Herstellung von Verbindungen mit zwei oder mehr Vinylsulfon-Gruppen
Procédé pour la fabrication des composés contenant deux ou plusieurs groupements vinylsulfones

(30) Priority: 30.08.1993 US 114035
(43) Date of publication of application: 01.03.1995
(73) Proprietor: EASTMAN KODAK COMPANY, Rochester, New York 14650 (US)
(72) Inventor: Kim, Chang-Kyu, c/o Eastman Kodak Co., Rochester, New York 14650-2201 (US); Gathmann, Linda Marie, c/o Eastman Kodak Co., Rochester, New York 14650-2201 (US); Osypian, Marc Alfred, c/o Eastman Kodak Co., Rochester, New York 14650-2201 (US)
(74) Representative: Wibbelmann, Jobst, Dr., Dipl.-Chem.

(56) References cited:
- US-A- 4 171 976

## Description

This invention relates to an improved process for the preparation of compounds containing two or more vinyl sulfone groups.

Compounds containing two or more vinyl sulfone groups (hereafter "vinyl sulfones") are well known cross-linking agents for hydrophilic colloids, such as photographic gelatin. These compounds are usually prepared from the corresponding haloethylsulfonyl compounds by dehydrohalogenation using a strong organic base such as triethylamine. One disadvantage that has been observed in making and using these vinyl sulfones is that these compounds, particularly the more active ones, such as bis-(vinylsulfonyl)methane (BVSM), will readily homopolymerize in the presence of a strong base. In U.S. Patent 4,171,976 to D.M. Burness et al it is disclosed that homopolymerization inhibitors, such as 3,5-dinitrobenzoic acid, can be used to prevent homopolymerization of the vinyl sulfones. However, even with such inhibitors, vinyl sulfones readily polymerize so that in the process of making a vinyl sulfone with a strong base, such as triethylamine, careful handling is required to prevent the presence of too much base. It is not easy to determine the exact stoichiometry in this process particularly because the quality of the precursor is variable and there is no good analytical method available for it. This results in commercial processes which are complicated and tedious and give unpredictable yields.

### Problem to be Solved by the Invention

This invention solves the problem of undesired homopolymerization of a compound having two or more vinyl sulfone groups during the manufacture thereof.

### Summary of the Invention

This invention comprises a method of preparing a compound of the formula:

(CH₂=CH-SO₂)ₙ-Z

by reacting a compound of the formula:

(CHX-CH₂-SO₂)ₙ-Z

where X is a halogen atom, Z is an organic radical having a valence of n and n is 2-6, in the presence of an alkali metal salt of a carboxylic acid. Use of the alkali metal salt of a carboxylic acid in the dehydrohalogenation reaction inhibits the tendency of the vinyl sulfone to homopolymerize during the manufacture thereof.

### Advantageous Effect of the Invention

The use of a weak base, such as an alkali metal salt of a carboxylic acid, provides an appropriate environment for the process since the by-product carboxylic acid acts as a buffer. In other words, even with excess of base, the reaction medium does not go over a certain pH because of carboxylic acid formed as by-product. Therefore, a small excess of base can be used to complete the reaction without having any danger of homopolymerization. Since the reaction is complete, the work-up is straight forward and the yield does not suffer. Furthermore, the by-products are only alkali metal halide and carboxylic acid. After removal of those by-products, the product does not require any further purification to meet the desired quality specifications. As a result, the process of this invention reduces solvent usage and minimizes waste streams.

### Detailed Description of the Invention

The process of this invention produces a vinyl sulfone of the formula:

(CH₂=CH-SO₂)ₙ-Z

where Z is an organic radical having a valence of n and n is 2-6.

In the above formula Z is preferably

-A-

-O-A-O- ,

or

-D-

where A is an alkylene group containing 1 to 8 carbon atoms which may be unsubstituted or substituted and the alkylene chain may be interrupted by one or more hetero atoms or organic groups, or an arylene group, which may be substituted or unsubstituted, and D is a trivalent alkylene group, a trivalent arylene group which may be substituted with one or more additional CH₂=CH-SO₂-groups, a trivalent cyclic alkylene group which may be substituted with one or more CH₂=CH-SO₂- groups, or a trivalent heterocyclic group which may be substituted with one or more CH₂=CH-SO₂- groups. Preferred substituents for A include -OH, phenyl, aralkyl, such as phenethyl, or CH₂=CH-SO₂- groups. The aryl moiety of the aralkyl group may be sulfonated. The alkylene group may be interrupted by one or more of the following: oxygen atoms, arylene groups, cycloalkyl groups, -NHCONH- , or -N-R, where R is an alkyl group containing 1 to 8 carbon atoms,

Illustrative vinyl sulfones are compounds having the following formulas:

CH₂=CHSO₂CH₂SO₃CH=CH₂

CH₂=CHSO₂CH₂CH₂SO₂CH=CH₂

CH₂=CHSO₂(CH₂)₅SO₂CH=CH₂

CH₂=CHSO₂OCH₂OSO₂CH=CH₂

CH₂=CHSO₂CH₂CH₂OCH₂CH₂NHCONHCH₂CH₂-OCH₂CH₂SO₂CH=CH₂

CH₂=CHSO₂CH₂CH₂OCH₂CH₂OCH₂CH₂S0₂CH=CH₂

CH₂=CHSO₂CH₂CH₂OCH₂CH₂CH₂CH₂OCH₂CH₂SO₂CH=CH₂

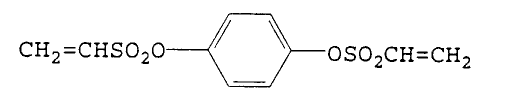

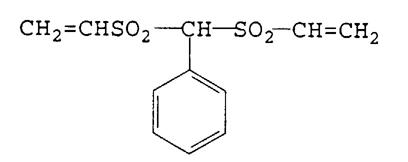

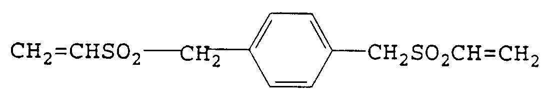

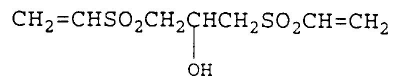

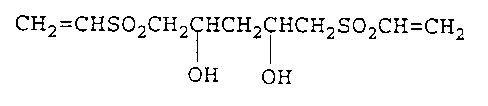

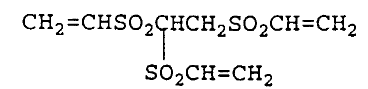

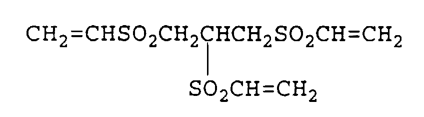

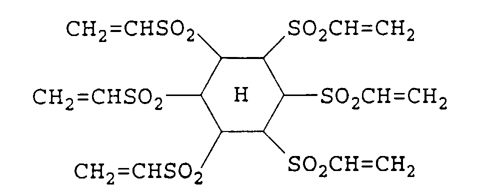

In a preferred embodiment, this invention provides the process for the preparation of a bis-(vinylsulfonyl)alkane having the formula:

CH₂=CH-SO₂-(CH₂)ₘ-SO₂-CH=CH₂ (I)

from a bis-(haloethylsulfonyl)alkane having the formula:

X-CH₂-CH₂-SO₂-(CH₂)ₘ-SO₂-CH₂-CH₂-X (II)

wherein X is a halogen, such as chlorine or bromine, and m is 1, 2, or 3; using an alkali metal salt, preferably a sodium or potassium salt of a carboxylic acid, preferably a low molecular weight carboxylic acid having the formula: 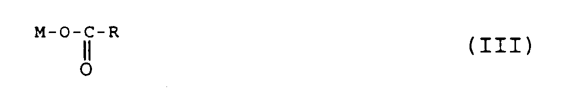 wherein M is an alkali metal, preferably sodium or potassium, and R is -H, or a substituted or unsubstituted hydrocarbyl group containing 1 to 7 carbon atoms.

The process of this preferred embodiment of the invention comprises dissolving compound II in an inert solvent and reacting with a slight excess of the sodium or potassium salt of a low molecular weight carboxylic acid (III) to produce desired compound I, sodium or potassium halide, and the carboxylic acid.

The process is characterized by clean and complete reaction without danger of homopolymerization of the compound I.

For the purpose of this invention, a sodium or potassium salt of low molecular weight carboxylic acid is characterized by having the following characteristics:
1. it is a weak base which does not induce homopolymerization of the compound I, but is strong enough to effect dehydrohalogenation.
2. its by-product, carboxylic acid, remains in solution while the reaction is in progress and is easily removable after the reaction is complete either by evaporation at low temperature or by washing with solvent(s). If the carboxylic acid is removed by evaporation, the temperature should be relatively low, for example in the range of 0 to 50°C under a reduced pressure of 0.1 to 100 Torr, since higher temperatures may cause homopolymerization of the vinyl sulfone even in the acidic reaction medium.
3. it is low cost and readily available.

In view of these desired characteristics, the carboxylic acid is defined as set forth above in formula (III), wherein R is H, or a hydrocarbyl group containing 1 to about 7 carbon atoms. The hydrocarbyl group may be substituted or unsubstituted. Examples are: sodium or potassium formate, acetate, propionate, butyrate, isobutyrate, pentanoate, hexanoate, heptanoate, octanoate, chloroacetate, bromoacetate, hydroxyacetate, benzoate, chlorobenzoate, and hydroxybenzoate. Among these, most preferred are sodium or potassium acetate and sodium or potassium propionate.

The amount of alkali metal salt of a carboxylic acid used in the dehydrohalogenation reaction should be a slight excess to make sure the reaction is complete. Generally, the amount of salt used should be 1% to 10% in excess of that required to effect complete dehydrohalogenation.

The inert solvent for the process of this invention comprises any solvent which does not react with either the starting material compound (II) or the product compound (I). A preferred solvent for the process of this invention is low cost and has low boiling point since it should be easily removable after its use by evaporation at a low temperature. Suitable solvents include, for example, acetone, methyl acetate, ethyl acetate, propyl acetate, diethyl ether, diisopropyl ether, tetrahydrofuran, acetonitrile, propionitrile, butyronitrile, chloroform, dichloromethane, dichloroethane, trichloroethane, benzene, and toluene.

The dehydrohalogenation reaction is generally conducted at a temperature which gives a reasonable rate of reaction, but which does not cause unwanted homopolymerization or extraneous side reactions with loss of yield of desired product. For example, temperatures of from 20°C to 60°C can be employed; generally it is preferred to use a temperature of from 30°C to 45°C.

Ambient pressures are preferred; however, somewhat reduced pressures can be used since it may help maintain the desired temperature by evaporation and reflux of solvent.

The reaction time is dependent upon the choice of salt and the reaction temperature. It should be neither too short since it may cause incomplete reaction, nor too long since it may cause homopolymerization or other side reactions. In general, reaction times of from 2 to 8 hours are preferred.

After the dehydrohalogenation reaction is complete, the excess of base may be acidified using a corresponding excess amount of hydrochloric acid or hydrobromic acid. Then, the resulting salt, sodium or potassium halide, can be removed by filtration or water washes. The product pure enough to meet the desired quality specifications can be obtained by known techniques, such as simple removal of solvent by distillation or crystallization from appropriate solvent or solvent mixture.

The following examples illustrate the preparation of a vinyl sulfone using the process of this invention.

### Example 1

A solution of 26.9 g (0.10 mol) of bis-(2-cholorethylsulfonyl)-methane and 0.1 g of 3,5-dinitrobenzoic acid in 70 ml of acetone is stirred with 19.8 g (0.206 mol) of sodium propionate at 40°C for 3 hours. The reaction mixture is cooled to 20°C and 5.3 ml (0.06 mol) of hydrochloric acid is added with stirring. The mixture is filtered to remove salt and to the filtrate is added 0.2 g of 3,5-dinitrobenzoic acid. The solution is concentrated to an oil under a reduced pressure removing solvent and most of propionic acid while keeping the temperature below 50°C. The oil is then crystallized from 35 ml of ethyl acetate and 70 ml of heptane to give 18.6 g (95%) of bis-(vinylsulfonyl) methane.

### Example 2

The same process described in Example 1 is carried out using 16.9 g (0.206 mol) of sodium acetate instead of sodium propionate. After removal of salt, the filtrate is concentrated to an oil under a reduced pressure removing solvent and acetic acid while keeping the pot temperature below 50°C. The oil is solidified upon cooling to give 19.4 g (99%) of pure bis-(vinylsulfonyl)methane meeting all quality specifications.

To compare the process of this invention with the existing one, the latter is described in the following example.

### Example 3 (Comparative)

This example illustrates a typical prior art process in which a strong base, triethyl amine, is used in the dehydrohalogenation reaction.

A solution of 26.9 g (0.10 mol) of bis-(2-chloroethylsulfonyl)-methane in 70 ml of acetone is treated 50°C with 5 g of activated carbon. The carbon is filtered off with filter aid and to the filtrate is added 0.1 g of 3,5-dinitrobenzoic acid. To the mixture is added at 35°C slowly 17.2 g (0.17 mol) of triethylamine. In-process LC monitoring unit is started and the slow addition of triethylamine is continued until the LC unit indicates 98 A% of desired product. It usually takes another 2 g of triethylamine which comprises total 0.95 equivalent of triethylamine. At this time, the reaction mixture is somewhat dark color so that it requires another carbon treatment. The triethylamine hydrochloride and carbon are removed by filtration and to the filtrate is added 0.2 g of 3,5-dinitrobenzoic acid. The mixture is then concentrated to an oil under a reduced pressure removing solvent while keeping the pot temperature under 50°C. The resulting oil is crystallized from 100 ml of methanol to give 15.1 g (77%) of product.

The vinyl sulfone compounds manufactured in accordance with this invention can be used as a hardener for gelatin, in particular gelatin in a photographic element. The use of vinylsulfones to harden gelatin in photographic elements is disclosed, for example, in U.S. Patents Nos. 3,490,911 to Burness et al, 3,841,872 to Burness et al, 4,171,976 to Burness et al, 4,057,538 to Habu et al, 4,088,495 to Habu et al, 4,168,172 to Kataoka et al, 4,173,481 to Sera et al and 4,897,344 to Okamura et al, the disclosures of which are incorporated herein by reference.

The invention has been described above with particular reference to preferred embodiments thereof.

## Claims

1. A method of preparing a vinyl sulfone compound of the formula:
(CH₂=CH-SO₂)ₙ-Z
by dehydrohalogenating a compound of the formula:
(CHX-CH₂-SO₂)ₙ-Z
where X is a halogen atom, Z is an organic radical having the valence n and n is 2-6, characterized in that said dehydrohalogenization is carried out in the presence of an alkali metal salt of a carboxylic acid.

2. The process of claim 1, wherein said alkali metal salt of a carboxylic acid has the formula: 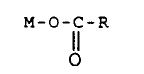 wherein M is sodium or potassium and R is -H, or a substituted or unsubstituted hydrocarbyl group containing 1 to 7 carbon atoms.

3. The process of claims 1 or 2, wherein said alkali metal salt of a carboxylic acid is sodium acetate or potassium acetate.

4. The process of claims 1 or 2, wherein said alkali metal salt of a carboxylic acid is sodium propionate or potassium propionate.

5. The process of any of claims 1 to 4, wherein the amount of said alkali metal salt of a carboxylic acid is 1% to 10% in excess of theoretical amount.

6. The process of claim 1 wherein said vinyl sulfone compound is a bis-(vinylsulfonyl)alkane having the formula:
CH₂=CH-SO₂-(CH₂)ₘ-SO₂-CH=CH₂
and is prepared by dehydrohalogenating a bis-(haloethylsulfonyl)alkane having the formula:
X-CH₂-CH₂-SO₂-(CH₂)ₘ-SO₂-CH₂-CH₂-X
wherein X is a halogen and m is 1, 2, or 3 in the presence of an alkali metal salt of a carboxylic acid having the formula: 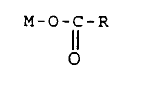 wherein M is sodium or potassium and R is -H or a substituted or unsubstituted hydrocarbyl group containing 1 to 7 carbon atoms.

7. The process of claim 1, wherein bis-(vinylsulfonyl)methane is prepared by dehydrohalogenating bis-(2-chloroethylsulfonyl)methane in the presence of sodium acetate or potassium acetate.

8. The process of claim 1, wherein bis-(vinylsulfonyl)methane is prepared by dehydrohalogenating bis-(2-chloroethylsulfonyl)methane in the presence of sodium propionate or potassium propionate.

## Patentansprüche

1. Verfahren zur Herstellung einer Vinylsulfonverbindung der Formel:
(CH₂=CH-SO₂)ₙ-Z
durch die Dehydrohalogenierung einer Verbindung der Formel:
(CHX-CH₂-SO₂)ₙ-Z
worin X für ein Halogenatom steht, Z ein organischer Rest mit der Wertigkeit n ist und n gleich 2 - 6 ist, dadurch gekennzeichnet, daß die Dehydrohalogenierung in Gegenwart eines Alkalimetallsalzes einer Carbonsäure durchgeführt wird.

2. Verfahren nach Anspruch 1, bei dem das Alkalimetallsalz einer Carbonsäure die Formel hat: 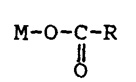 worin M für Natrium oder Kalium steht und R gleich -H oder eine substituierte oder unsubstituierte Hydrocarbylgruppe mit 1 bis 7 Kohlenstoffatomen ist.

3. Verfahren nach Ansprüchen 1 oder 2, bei dem das Alkalimetallsalz einer Carbonsäure Natriumacetat oder Kaliumacetat ist.

4. Verfahren nach Ansprüchen 1 oder 2, bei dem das Alkalimetallsalz einer Carbonsäure Natriumpropionat oder Kaliumpropionat ist.

5. Verfahren nach einem der Ansprüche 1 bis 4, bei dem die Menge an dem Alkalimetallsalz einer Carbonsäure bei einem 1- bis 10%igen Überschuß der theoretischen Menge liegt.

6. Verfahren nach Anspruch 1, bei dem die Vinylsulfonverbindung ein Bis-(vinylsulfonyl)alkan der Formel ist:
CH₂=CH-SO₂-(CH₂)ₘ-SO₂-CH-CH₂
und hergestellt wird durch Dehydrohalogenierung eines Bis-(haloethylsulfonyl)alkans mit der Formel:
X-CH₂-CH₂-SO₂-(CH₂)ₘ-SO₂-CH₂-CH₂-X
worin X für ein Halogenatom steht und m gleich 1, 2 oder 3 ist, in Gegenwart eines Alkalimetallsalzes einer Carbonsäure der Formel: 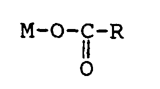 worin M für Natrium oder Kalium steht und R die Bedeutung von -H oder einer substituierten oder unsubstituierten Hydrocarbylgruppe mit 1 bis 7 Kohlenstoffatomen hat.

7. Verfahren nach Anspruch 1, bei dem das Bis-(vinylsulfonyl)-methan hergestellt wird durch Dehydrohalogenierung von Bis-(2-chloroethylsulfonyl)methan in Gegenwart von Natriumacetat oder Kaliumacetat.

8. Verfahren nach Anspruch 1, bei dem das Bis-(vinylsulfonyl)-methan hergestellt wird durch Dehydrohalogenierung von Bis-(2-chloroethylsulfonyl)methan in Gegenwart von Natriumpropionat oder Kaliumpropionat.

## Revendications

1. Procédé de préparation d'un composé vinylsulfone répondant à la formule :
(CH₂=CH-SO₂)ₙ-Z
par déshydrohalogénation d'un composé répondant à la formule :
(CHX-CH₂-SO₂)ₙ-Z
où X est un atome d'halogène, Z est un radical organique ayant la valence n et n est compris entre 2 et 6, caractérisé en ce que ladite déshydrohalogénation est effectuée en présence d'un sel de métal alcalin d'un acide carboxylique.

2. Procédé selon la revendication 1, dans lequel ledit sel de métal alcalin d'un acide carboxylique répond à la formule : 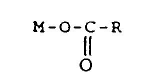 où M est un sodium ou un potassium et R est -H ou un groupe hydrocarbyle substitué ou non, contenant 1 à 7 atomes de carbone.

3. Procédé selon l'une des revendications 1 ou 2, dans lequel ledit sel de métal alcalin d'un acide carboxylique est un acétate de sodium ou un acétate de potassium.

4. Procédé selon l'une des revendications 1 ou 2, dans lequel ledit sel de métal alcalin d'un acide carboxylique est un propionate de sodium ou un propionate de potassium.

5. Procédé selon l'une quelconque des revendications 1 à 4, dans lequel la quantité dudit sel de métal alcalin d'un acide carboxylique est supérieure de 1 % à 10 % à la quantité théorique.

6. Procédé selon la revendication 1, dans lequel ledit composé vinylsulfone est un bis-(vinylsulfonyl)alcane répondant à la formule :
CH₂=CH-SO₂-(CH₂)ₘ-SO₂-CH=CH₂
et est préparé par déshydrohalogénation d'un bis-(haloéthylsulfonyl)alcane répondant à la formule :
X-CH₂-CH₂-SO₂-(CH₂)ₘ-SO₂-CH₂-CH₂-X
où X est un halogène et m est 1, 2 ou 3, en présence d'un sel de métal alcalin d'un acide carboxylique répondant à la formule : 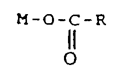 où M est un sodium ou un potassium et R est -H ou un groupe hydrocarbyle substitué ou non, contenant 1 à 7 atomes de carbone.

7. Procédé selon la revendication 1, dans lequel du bis-(vinylsulfonyl)méthane est préparé par déshydrohalogénation de bis-(2-chloroéthylsulfonyl)méthane en présence d'acétate de sodium ou d'acétate de potassium.

8. Procédé selon la revendication 1, dans lequel du bis-(vinylsulfonyl)méthane est préparé par déshydrohalogénation de bis-(2-chloroéthylsulfonyl)méthane en présence de propionate de sodium ou de propionate de potassium.
